(19) Europäisches Patentamt
European Patent Office
Office européen des brevets

(11) Veröffentlichungsnummer: **0 022 511**
**B1**

# (12) EUROPÄISCHE PATENTSCHRIFT

(45) Veröffentlichungstag der Patentschrift: **09.03.83**

(51) Int. Cl.³: **C 07 D239/52**, C 07 D239/56, A 01 N 47/18

(21) Anmeldenummer: **80103720.1**

(22) Anmeldetag: **01.07.80**

## (54) N,N-Dimethyl-O-pyrimidinyl-carbaminsäureester, Verfahren zu ihrer Herstellung sowie ihre Verwendung als Schädlingsbekämpfungsmittel.

(30) Priorität: **12.07.79 DE 2928185**

(43) Veröffentlichungstag der Anmeldung: **21.01.81 Patentblatt 81/03**

(45) Bekanntmachung des Hinweises auf die Patenterteilung: **09.03.83 Patentblatt 83/10**

(84) Benannte Vertragsstaaten: **AT BE CH DE FR GB IT LI NL SE**

(56) Entgegenhaltungen:
**EP A 0 002 200**
**CH A 281 966**
**FR A 1 443 910**

**Die Akte enthält technische Angaben, die nach dem Eingang der Anmeldung eingereicht wurden und die nicht in dieser Patentschrift enthalten sind.**

(73) Patentinhaber: **BAYER AG**
**Zentralbereich Patente, Marken und Lizenzen**
**D-5090 Leverkusen 1, Bayerwerk (DE)**

(72) Erfinder: **Maurer, Fritz, Dr.**
**Roeberstrasse 8**
**D-5600 Wuppertal 1 (DE)**
Erfinder: **Hammann, Ingeborg, Dr.**
**Belfortstrasse 9**
**D-5000 Köln 1 (DE)**
Erfinder: **Homeyer, Bernhard, Dr.**
**Obere Strasse 28**
**D-5090 Leverkusen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 022 511 B1

N,N-Dimethyl-O-pyrimidinyl-carbaminsäureester, Verfahren zu ihrer Herstellung und ihre Verwendung als Schädlingsbekämpfungsmittel

Die Erfindung betrifft neue N,N-Dimethyl-O-pyrimidinyl-carbaminsäureester, mehrere Verfahren zu ihrer Herstellung sowie ihre Verwendung als Schädlingsbekämpfungsmittel, insbesondere als Insektizide.

Es ist bekannt, daß bestimmte N,N-Dialkyl-O-pyrimidinyl-carbaminsäureester, wie z. B. N,N-Dimethyl-O-(2-isopropyl-6-methyl-pyrimidin(4)yl)- und N,N-Dimethyl-O-(2-methylthio-6-methyl-pyrimidin(4)yl)-carbaminsäureester, insektizide Eigenschaften aufweisen (vergleiche FR-PS 1 443 910 und US-PS 2 694 712).

Die insektizide Wirkung dieser Verbindungen ist jedoch, insbesondere bei niedrigen Wirkstoffkombinationen und Aufwandmengen, nicht immer zufriedenstellend.

1. Gegenstand der Erfindung sind neue N,N-Dimethyl-O-pyrimidinyl-carbaminsäureester der Formel I

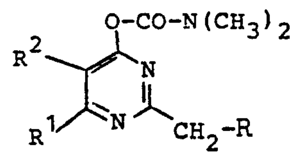

(I)

in welcher

R und $R^2$ gleich oder verschieden sind und einzeln für Alkoxy, Alkylthio, Alkylsulfinyl oder Alkylsulfonyl mit jeweils 1-6 C-Atomen stehen, und

$R^1$ für Wasserstoff oder Alkyl mit 1-6 C-Atomen steht.

2. Verfahren zur Herstellung der neuen N,N-Dimethyl-O-pyrimidinyl-carbaminsäureester der Formel I, dadurch gekennzeichnet, daß man

a) Hydroxy-pyrimidine der Formel II

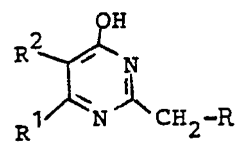

(II)

in welcher R, $R^2$ und $R^1$ die oben angegebenen Bedeutungen haben mit N,N-Dimethyl-carbaminsäurehalogeniden der Formel III

$$Hal{-}CO{-}N(CH_3)_2 \qquad (III)$$

in welcher Hal für Chlor oder Brom steht,
gegebenenfalls in Gegenwart eines Säureakzeptors und gegebenenfalls unter Verwendung eines Verdünnungsmittels umsetzt, oder

b) Hydroxy-pyrimidine der Formel II, worin R, $R^2$ und $R^1$ die oben angegebenen Bedeutungen haben, mit Phosgen und anschließend mit Dimethylamin, gegebenenfalls in Gegenwart eines Säureakzeptors und gegebenenfalls unter Verwendung eines Verdünnungsmittels umsetzt.

Verbindungen der Formel I, in welcher R und/oder $R^2$ für Alkylsulfinyl steht, erhält man auch, indem man

c) N,N-Dimethyl-O-pyrimidinyl-carbaminsäureester der Formel I, in welcher R und/oder $R^2$ für Alkylthio steht, mit der äquimolaren Menge Wasserstoffperoxid, gegebenenfalls unter Verwendung eines Verdünnungsmittels umsetzt.

Verbindungen der Formel I in welcher R und/oder $R^2$ für Alkylsulfonyl steht, erhält man auch, indem man

d) N,N-Dimethyl-O-pyrimidinyl-carbaminsäureester der Formel I, in welcher R und/oder $R^2$ für Alkylthio steht, mit wenigstens zwei Moläquivalenten m-Chlorperbenzoesäure, gegebenenfalls in Gegenwart eines Verdünnungsmittels umsetzt.

Die Hydroxypyrimidine der Formel II

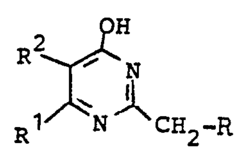

(II)

in welcher R, $R^1$, $R^2$ die unter 1) angegebene Bedeutung haben sind neu.

Sie werden hergestellt, indem man

a) Alkoxy- oder Alkylthioessigsäureester der Formel IV

$$R^2—CH_2—COO\ R^3 \qquad (IV)$$

in welcher

$R^2$ die oben angegebene Bedeutung besitzt und

$R^3$ für Alkyl mit 1-4 C-Atomen steht

mit Estern der Formel V

$$R^1—COOR^4 \qquad (V)$$

in welcher

$R^1$ die unter 1) angegebene Bedeutung besitzt und

$R^4$ für Alkyl mit 1-4 C-Atomen steht,

sowie mit Alkoxy- oder Alkylthioacetamidinen der Formel VI

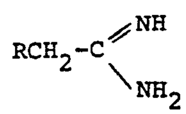

(VI)

in welcher R die unter 1) angegebene Bedeutung besitzt,

umsetzt, oder

b) 5-Alkoxy- oder 5-Alkylthio-2-chlormethyl-6-hydroxypyrimidine der Formel VII

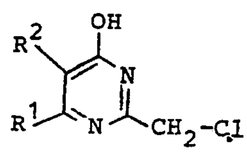

(VII)

in welcher $R^2$ und $R^1$ die unter 1) angegebene Bedeutung besitzen

mit Alkalialkoholaten oder -mercaptiden der Formel VIII

$$R'—X—Alkali$$

in welcher

R′ für $C_{1-4}$Alkyl steht,

X für O oder S steht und

Alkali für ein Alkaliion steht,

umsetzt.

Die neuen N,N-Dimethyl-O-pyrimidinyl-carbaminsäureester der Formel I zeichnen sich durch hohe Wirksamkeit als Schädlingsbekämpfungsmittel, insbesondere durch hervorragende insektizide Wirkung aus.

Überraschenderweise zeigen die erfindungsgemäßen N,N-Dimethyl-O-pyrimidinyl-carbaminsäureester eine erheblich höhere insektizide Wirkung als aus dem Stand der Technik bekannten Verbindungen analoger Konstitution und gleicher Wirkungsrichtung.

Gegenstand der Erfindung sind vorzugsweise Verbindungen der Formel I, in welcher

R und $R^2$ gleich oder verschieden sind und einzeln für Alkoxy, Alkylthio, Alkylsulfinyl oder Alkylsulfonyl mit 1 bis 3 Kohlenstoffatomen stehen, und

$R^1$ für Wasserstoff oder Alkyl mit 1 bis 4 Kohlenstoffatomen steht.

Verwendet man beispielsweise bei Verfahren (a) N,N-Dimethyl-carbaminsäurechlorid, bei Verfahren (b) Phosgen und Dimethylamin, und bei beiden Verfahren 2-Ethoxymethyl-4-iso-propyl-5-methylthio-6-hydroxy-pyrimidin, bei Verfahren (c) N,N-Dimethyl-O-(2-methylthiomethyl-4-tert.-butyl-5-iso-propoxy-pyrimidin-6-yl)-carbaminsäureester und Wasserstoffperoxid, und bei Verfahren (d) N,N-Dimethyl-O-(2-methoxymethyl-5-methylthiopyrimidin-6-yl)-carbaminsäureester und m-Chlorperbenzoesäure als Ausgangstoffe, so können die entsprechenden Reaktionen durch folgende Formelschemata skizziert werden :

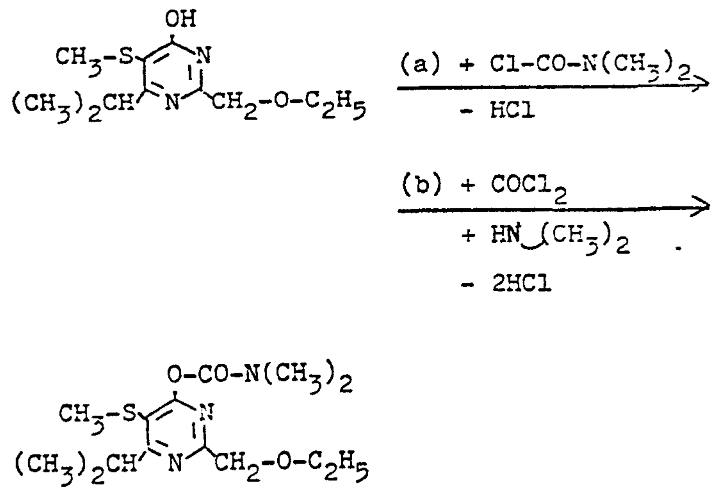


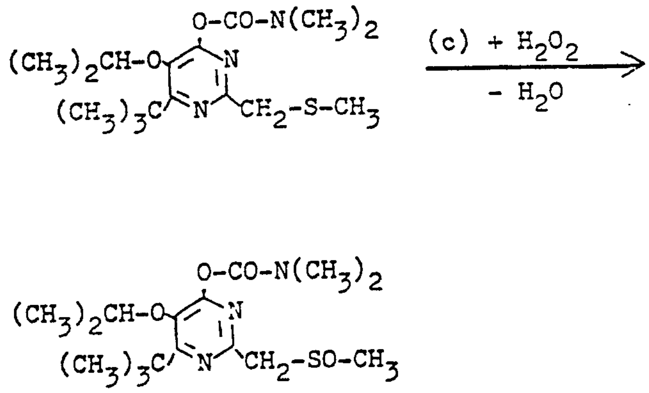


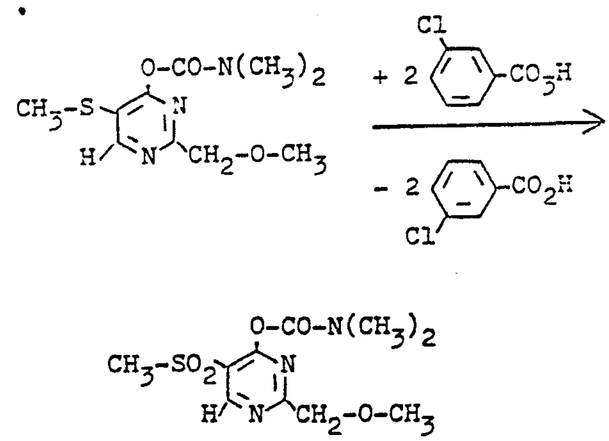


Die bei den Verfahren (a) und (b) als Ausgangsstoffe zu verwendenden Hydroxy-pyrimidine sind durch Formel II definiert. Vorzugsweise stehen darin R, $R^2$ und $R^1$ für diejenigen Reste, welche bei der Definition der Reste R, $R^2$ und $R^1$ in Formel I als bevorzugt genannt wurden.

Als Beispiel für die Verbindungen der Formel II seien genannt :

2-Methylthiomethyl-5-methoxy-, 2-Methoxymethyl-5-methoxy-, 2-Äthylthiomethyl-5-methoxy-, 2-n-Propylthiomethyl-5-methoxy-, 2-Methylthiomethyl-5-äthoxy-, 2-Methylthiomethyl-5-methylthio-, 2-Methylthiomethyl-5-iso-propoxy-, 2-Methylthiomethyl-5-äthoxy-, 2-Methylthiomethyl-4-methyl-5-methylthio-, 2-Methylthiomethyl-4-äthyl-5-methylthio-, 2-Methylthiomethyl-4-tert.-butyl-5-methylthio-, 2-iso-Propylthiomethyl-5-methoxy-, 2-Methylthiomethyl-5-n-propoxy-, 2-Äthylthiomethyl-5-äthoxy-, 2-Methylthiomethyl-5-äthylthio- und 2-Äthylthiomethyl-5-äthoxy-6-hydroxy-pyrimidin

Die Hydroxy-pyrimidine der Formel II sind noch nicht in der Literatur beschrieben. Man erhält diese

Verbindungen :

a) durch Umsetzung von Alkoxy- oder Alkylthio-essigsäureestern, beispielsweise von Methoxyessigsäuremethylester, mit Estern, wie z. B. Ameisensäure-methylester, in Gegenwart von Basen, wie z. B. Natriummethylat, und gegebenenfalls unter Verwendung von Verdünnungsmitteln, wie z. B. Methanol, bei Temperaturen zwischen 0 und 50 °C, und Umsetzung der hierbei erhaltenen Produkte mit Alkoxy- oder Alkylthio-acetamidinen oder deren Hydrochloriden, wie z. B. Methylthio-acetamidin-hydrochlorid, ebenfalls bei Temperaturen zwischen 0 und 50 °C. Zur Aufarbeitung wird das Lösungsmittel im Vakuum abgezogen, der Rückstand in Wasser gelöst und ein pH-Wert von ungefähr 6 eingestellt. Man extrahiert mit einem mit Wasser nicht mischbaren Lösungsmittel, wie z. B. Methylenchlorid, und destilliert vom Extrakt das Lösungsmittel ab, wobei das Produkt als fester oder öliger Rückstand erhalten wird. Alkylthiomethyl-hydroxy-pyrimidine der Formel II können mit Oxidationsmitteln, wie z. B. Wasserstoffperoxid oder m-Chlor-perbenzoesäure, bei Temperaturen zwischen 0 und 50 °C, gegebenenfalls unter Verwendung von Verdünnungsmitteln, wie z. B. Essigsäure oder Chloroform, zu den entsprechenden Alkylsulfinylmethyl- bzw. Alkylsulfonyl-methyl-hydroxy-pyrimidinen oxidiert werden.

Hydroxy-pyrimidine der Formel II erhält man auch

b) durch Umsetzung von 5-Alkoxy- oder 5-Alkylthio-2-chlormethyl-6-hydroxy-pyrimidinen mit Alkalialkoholaten oder -mercaptiden bei Temperaturen zwischen 20 und 100 °C, gegebenenfalls unter Verwendung von Verdünnungsmitteln, wie z. B. Methanol oder Acetonitril. Zur Aufarbeitung wird ein pH-Wert von ungefähr 6 eingestellt und das Lösungsmittel abdestilliert. Der Rückstand wird mit Äthanol digeriert, die Suspension filtriert und das Filtrat durch Vakuumdestillation vom Lösungsmittel befreit. Man erhält so die Produkte in fester oder öliger Form.

Die als Vorstufen einzusetzenden 5-Alkoxy- oder 5-Alkylthio-2-chlormethyl-6-hydroxy-pyrimidine erhält man durch Umsetzung von Chloracetonitril mit Ammoniumchlorid in Gegenwart einer Base, wie z. B. Natriummethylat, gegebenenfalls unter Verwendung eines Verdünnungsmittels, wie z. B. Methanol, bei Temperaturen zwischen − 10 und + 30 °C und anschließende Umsetzung der hierbei erhaltenen Produkte mit den durch Umsetzung von Alkoxy- bzw. Alkylthioessigsäureestern mit Ameisensäureestern in Gegenwart von Basen, wie z. B. Natriummethylat, erhaltenen Produkten ebenfalls bei Temperaturen zwischen − 10 und + 30 °C und gegebenenfalls in Gegenwart von Basen, wie z. B. Natriummethylat, gegebenenfalls unter Verwendung von Verdünnungsmitteln, wie z. B. Methanol. Zur Aufarbeitung stellt man den pH-Wert auf ungefähr 5 ein, verdünnt mit Wasser und destilliert gegebenenfalls das organische Lösungsmittel ab. Das Produkt kristallisiert aus der wässrigen Lösung aus.

Als Beispiel für die bei Verfahren a) zu verwendenden Carbaminsäurehalogenide der Formel III sei N,N-Dimethyl-carbaminsäurechlorid genannt. Es ist, ebenso wie die bei Verfahren b) einzusetzenden Reaktionskomponenten Phosgen und Dimethylamin, lange bekannt.

Die bei den Verfahren c) und d) als Ausgangsverbindungen zu verwendenden N,N-Dimethyl-O-pyrimidinyl-carbaminsäureester sind durch die Formel I mit der Maßgabe, daß wenigstens einer der Reste R oder $R^2$ für Alkylthio steht, definiert.

Vorzugsweise stehen darin R, $R^2$ und $R^1$ für diejenigen Reste, welche bei der Definition der Reste R, $R^2$ und $R^1$ in Formel I als bevorzugt genannt wurden.

Als Beispiele für die als Ausgangsverbindungen verwendbaren Verbindungen der Formel I seien genannt :

O-(2-Methylthiomethyl-5-methoxy-pyrimidin-6-yl)-,
O-(2-Methylthiomethyl-5-äthoxy-pyrimidin-6-yl)-,
O-(2-Äthylthiomethyl-5-methoxy-pyrimidin-6-yl)-,
O-(2-n-Propylthiomethyl-5-methoxy-pyrimidin-6-yl)-,
O-(2-Methylthiomethyl-5-iso-propoxy-pyrimidin-6-yl)-,
O-(2-Methylthiomethyl-5-methylthio-pyrimidin-6-yl)-,
O-(2-Methylthiomethyl-5-äthylthio-pyrimidin-6-yl)-,
O-(2-Methylthiomethyl-4-methyl-5-methylthio-pyrimidin-6-yl)-,
O-(2-Methylthiomethyl-4-äthyl-5-methylthio-pyrimidin-6-yl)-,
O-(2-Methylthiomethyl-4-tert.-butyl-5-methylthio-pyrimidin-6-yl)-,
O-(2-Isopropylthiomethyl-5-methoxy-pyrimidin-6-yl)-,
O-(2-Methylthiomethyl-5-n-propoxy-pyrimidin-6-yl)-, und
O-2(Äthylthiomethyl-5-äthoxy-pyrimidin-6-yl)
-N,N-dimethylcarbaminsäureester.

Die bei Verfahren c) bzw. d) zu verwendenden Oxidationsmittel Wasserstoffperoxid bzw. m-Chlor-perbenzoesäure sind bekannte Verbindungen.

Die Verfahren a) bis d) zur Herstellung der neuen N,N-Dimethyl-O-pyrimidinyl-carbaminsäureester werden im allgemeinen unter Verwendung von Verdünnungsmitteln durchgeführt. Als solche kommen praktisch alle inerten organischen Lösungsmittel in Frage. Hierzu gehören insbesondere aliphatische und aromatische, gegebenenfalls chlorierte Kohlenwasserstoffe, wie Benzin, Benzol, Toluol, Xylol, Methylenchlorid, Chloroform, Tetrachlorkohlenstoff, Chlorbenzol und o-Dichlorbenzol, Äther wie Diäthyl-und Dibutyläther, Tetrahydrofuran und Dioxan, Ketone wie Aceton, Methyläthyl-, Methylisopropyl- und Methylisobutylketon sowie Nitrile wie Acetonitril und Propionitril.

Verfahren c) wird vorteilhaft unter Verwendung aliphatischer Carbonsäuren, wie z. B. Ameisensäure, Essigsäure oder Propionsäure, als Verdünnungsmittel durchgeführt.

Verfahren a) und b) werden im allgemeinen unter Verwendung von Säureakzeptoren durchgeführt. Als Säureakzeptoren können alle üblichen Säurebindemittel Verwendung finden. Besonders bewährt haben sich Alkalicarbonate und -alkoholate, wie Natrium- und Kaliumcarbonat, Natrium- und Kaliummethylat bzw. -äthylat, ferner aliphatische, aromatische oder heterocyclische Amine, beispielsweise Triäthylamin, Trimethylamin, Dimethylanilin, Dimethylbenzylamin und Pyridin.

Die erfindungsgemäßen Verfahren werden im allgemeinen bei Temperaturen zwischen 0 und 150 °C durchgeführt. Bevorzugt wird für Verfahren a) der Temperaturbereich zwischen 20 und 100 °C, für Verfahren b), c) und d) der Bereich zwischen 0 und 50 °C. Die Umsetzungen werden im allgemeinen bei Normaldruck durchgeführt.

Zur Durchführung von Verfahren a) bzw. b) werden auf 1 Mol Hydroxy-pyrimidin der Formel II zwischen 1,0 und 1,3 vorzugsweise zwischen 1,0 und 1,15 Mol N,N-Dimethylcarbamidsäurehalogenid bzw. Phosgen und Dimethylamin eingesetzt. Die Umsetzung wird im allgemeinen in einem Verdünnungsmittel in Gegenwart eines Säureakzeptors durchgeführt. Nach Ablauf der Reaktion wird filtriert und das Lösungsmittel im Vakuum abdestilliert.

Bei Verfahren c) werden Reaktionskomponenten in äquimolaren Mengen eingesetzt. Bei Verwendung von Verdünnungsmitteln, die mit Wasser mischbar sind, werden diese nach Reaktionsende im Vakuum abdestilliert. Der Rückstand wird dann in einem mit Wasser nicht mischbaren Lösungsmittel, wie z. B. Methylenchlorid, gelöst und nach üblichen Methoden, z. B. durch Waschen, Trocknen, Filtrieren und Abdestillieren des Lösungsmittels aufgearbeitet.

Bei Verfahren d) wird die als Oxidationsmittel verwendete m-Chlor-perbenzoesäure gewöhnlich im Überschuß eingesetzt, und zwar zwischen 2 und 3 Mol je Mol N,N-Dimethyl-O-(alkylthiomethyl-pyrimidinyl)-carbaminsäureester. Die Umsetzung wird im allgemeinen in einem mit Wasser nicht mischbaren Lösungsmittel durchgeführt. Nach Ablauf der Reaktion wird neutral gewaschen, getrocknet, filtriert und das Lösungsmittel im Vakuum abdestilliert.

Die neuen Verbindungen fallen in Form von Ölen an, die sich zum Teil nicht unzersetzt destillieren lassen, jedoch durch sogenanntes « Andestillieren », d. h. durch längeres Erhitzen unter vermindertem Druck auf mäßig erhöhte Temperaturen von den letzten flüchtigen Anteilen befreit und auf diese Weise gereinigt werden. Zu ihrer Charakterisierung dient der Brechungsindex.

Soweit die neuen Verbindungen nach dem Abdestillieren des Lösungsmittels in fester Form anfallen, werden sie durch Umkristallisieren gereinigt. Zur Charakterisierung dient dann der Schmelzpunkt.

Die erfindungsgemäßen N,N-Dimethyl-O-pyrimidinylcarbaminsäureester zeichnen sich durch hohe insektizide, insbesondere auch wurzelsystemische Wirksamkeit aus. Sie können daher zum Schutz von Nutzpflanzen gegen Insekten in Land- und Forstwirtschaft sowie im Hygienebereich und Vorratsschutz verwendet werden.

Die Wirkstoffe eignen sich bei guter Pflanzenverträglichkeit und günstiger Warmblütertoxizität zur Bekämpfung von tierischen Schädlingen, insbesondere Insekten, die in der Landwirtschaft, in Forsten, im Vorrats- und Materialschutz gewie auf dem Hygienesektor vorkommen. Sie sind gegen normal sensible und resistente Arten sowie gegen alle oder einzelne Entwicklungsstadien wirksam. Zu den oben erwähnten Schädlingen gehören :

Aus der Ordnung der Isopoda z. B. Oniscus asellus, Armadillidium vulgare, Porcellio scaber.

Aus der Ordnung der Diplopoda z. B. Blaniulus guttulatus.

Aus der Ordnung der Chilopoda z. B. Geophilus carpophagus, Scutigera spec.

Aus der Ordnung der Symphyla z. B. Scutigerella immaculata.

Aus der Ordnung der Thysanura z. B. Lepisma saccharina.

Aus der Ordnung der Collembola z. B. Onychiurus armatus.

Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z. B. Forficula auricularia.

Aus der Ordnung der Isoptera z. B. Reticulitermes spp..

Aus der Ordnung der Anoplura z. B. Phylloxera vastatrix, Pemphigus spp., Pediculus humanus corporis, Haematopinus spp., Linognathus spp.

Aus der Ordnung der Mallophaga z. B. Trichodectes spp., Damalinea spp.

Aus der Ordnung der Thysanoptera z. B. Hercinothrips femoralis, Thrips tabaci.

Aus der Ordnung der Heteroptera z. B. Eurygaster spp., Dysdercus intermedius, Piesma quadrata, Cimex lectularius, Rhodnius prolixus, Triatoma spp.

Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp.

Psylla spp.

Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z. B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z. B. Diprion spp., Hoplocampa spp., Lasius spp., Monomorium pharaonis, Vespa spp.

Aus der Ordnung der Diptera z. B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glykol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid ; als feste Trägerstoffe kommen in Frage : z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate ; als Dispergiermittel kommen in Frage : z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0, 000 000 1 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Beispiel A

Myzus-Test

Lösungsmittel : 3 Gewichtsteile Dimethylformamid
Emulgator : 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden ; 0 % bedeutet, daß keine Blattläuse abgetötet wurden.

Bei diesem Test zeigen z. B. die folgenden Verbindungen der Herstellungsbeispiele überlegene Wirksamkeit gegenüber dem Stand der Technik : 1,2,3,4,5,6.

Beispiel B

Doralis-Test (systemische Wirkung)

Lösungsmittel : 3 Gewichtsteile Dimethylformamid
Emulgator : 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit je 20 ml Wirkstoffzubereitung der gewünschten Konzentration werden Bohnenpflanzen (Vicia faba), die stark von der schwarzen Bohnenlaus (Doralis fabae) befallen sind, angegossen, so daß die Wirkstoffzubereitung in den Boden eindringt, ohne den Sproß zu benetzen. Der Wirkstoff wird von den Wurzeln aufgenommen und in den Sproß weitergeleitet.

Nach der gewünschten Zeit wird die Abtötung in % bestimmt. Dabei bedeutet 100 %, daß alle Blattläuse abgetötet wurden ; 0 % bedeutet, daß keine Blattläuse abgetötet wurden.

Bei diesem Test zeigen z. B. die folgenden Verbindungen der Herstellungsbeispiele überlegene Wirksamkeit gegenüber dem Stand der Technik : 2,3,4,5,6.

Beispiel C

Grenzkonzentrations-Test/Wurzelsystemische Wirkung

Testinsekt : Myzus persicae
Lösungsmittel : 3 Gewichtsteile Aceton
Emulgator : 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (=mg/1) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigt z. B. die Verbindung 1 der Herstellungsbeispiele überlegene Wirkung gegenüber dem Stand der Technik.

## Herstellungsbeispiele

### Beispiel 1

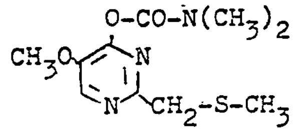

Ein Gemisch aus 18,6 g (0,1 Mol) 2-Methylthiomethyl-6-hydroxy-5-methoxypyrimidin, 16,6 g (0,12 Mol) Kaliumcarbonat, 200 ml Acetonitril und 10,8 g (0,1 Mol) Dimethylcarbaminsäurechlorid wird 7 Stunden unter Rückfluß gekocht, auf Raumtemperatur abgekühlt und dann filtriert. Das Filtrat wird im Vakuum eingedampft. Zurück bleiben 19,5 g (76 % der Theorie) N,N-Dimethyl-0-(2-methylthiomethyl-5-methoxypyrimidin-6-yl)-carbaminsäureester in Form beiger Kristalle mit dem Schmelzpunkt 97 °C.

### Beispiel 2

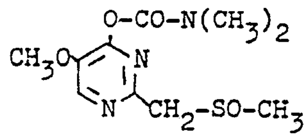

Zu einer Lösung von 25,7 g (0,1 Mol) N,N-Dimethyl-0-(2-methyl-thiomethyl-5-methoxy-pyrimidin-6-yl)-carbaminsäureester in 100 ml Eisessig tropft man bei 5-10 °C 11,2 g 30 proz. $H_2O_2$-Lösung in Wasser. Dann rührt man das Gemisch 18 Stunden bei Raumtemperatur nach, destilliert das Lösungsmittel im Vakkum ab und löst den Rückstand in 100 ml Methylenchlorid. Die Lösung wird einmal mit 40 ml 50 proz. Kaliumcarbonatlösung gewaschen, über Natriumsulfat getrocknet und im Vakuum eingedampft. Man erhält auf diese Weise 22,4 g (82 % der Theorie) N,N-Dimethyl-O-(2-methylsulfinylmethyl-5-methoxy-pyrimidin-6-yl)-carbaminsäure-ester in Form gelber Kristalle mit dem Schmelzpunkt 112 °C.

### Beispiel 3

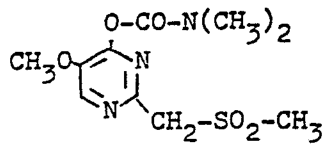

Eine Lösung von 25,7 g (0,1 Mol) N,N-Dimethyl-0-(2-methylthiomethyl-5-methoxypyrimidin-6-yl)-carbaminsäureester in 100 ml Chloroform wird bei 0-5 °C mit einer Lösung von 44,5 g (0,22 Mol) m-Chlor-perbenzoesäure in 500 ml Chloroform versetzt und dann 18 Stunden bei Raumtemperatur nachgerührt. Dann filtriert man vom Ungelösten und schüttelt das Filtrat mit 50 ml 50 proz. Kaliumcarbonat. Nach Trocknen über Natriumsulfat wird das Lösungsmittel im Vakuum abdestilliert. Zurück bleiben 28,3 g (95 % der Theorie) N,N-Dimethyl-O-(2-methylsulfonylmethyl-5-methoxypyrimidin-6-yl)-carbaminsäureester in Form farbloser Kristalle mit dem Schmelzpunkf 122 °C.

In analoger Weise können die folgenden Verbindungen der Formel

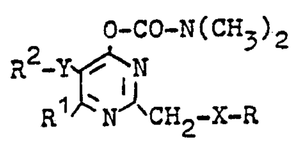

hergestellt werden

| Beisp. Nr. | R | $R^1$ | $R^2$ | X | Y | Ausb. (% d.Th.) | Brechungs-index Schmp.(°C) |
|---|---|---|---|---|---|---|---|
| 4 | $C_2H_5$ | H | $CH_3$ | S | 0 | 55 | 83 |
| 5 | $C_2H_5$ | H | $CH_3$ | SO | 0 | 97 | 116 |

(Fortsetzung)

| Beisp. Nr. | R | $R^1$ | $R^2$ | X | Y | Ausb. (% d.Th.) | Brechungsindex Schmp.(°C) |
|---|---|---|---|---|---|---|---|
| 6 | $C_2H_5$ | H | $CH_3$ | $SO_2$ | O | 94 | 113 |
| 7 | $n-C_3H_7$ | H | $CH_3$ | S | O | | |
| 8 | $CH_3$ | H | $C_2H_5$ | S | O | | |
| 9 | $CH_3$ | H | $i-C_3H_7$ | S | O | | |
| 10 | $CH_3$ | H | $CH_3$ | O | O | 70 | 75 |
| 11 | $CH_3$ | H | $CH_3$ | S | S | | |
| 12 | $CH_3$ | H | $C_2H_5$ | S | S | | |
| 13 | $CH_3$ | $CH_3$ | $CH_3$ | S | S | | |
| 14 | $CH_3$ | $C_2H_5$ | $CH_3$ | S | S | | |
| 15 | $CH_3$ | $t-C_4H_9$ | $CH_3$ | S | S | | |
| 16 | $CH_3$ | H | $CH_3$ | SO | SO | | |
| 17 | $CH_3$ | H | $CH_3$ | $SO_2$ | $SO_2$ | | |
| 18 | $iC_3H_7$ | H | $CH_3$ | S | O | | |
| 19 | $CH_3$ | H | $n-C_3H_7$ | S | O | | |
| 20 | $C_2H_5$ | H | $C_2H_5$ | S | O | | |

Die als Ausgangsmaterialien zu verwendenden Hydroxypyrimidine können z. B. wie folgt hergestellt werden :

Beispiel a

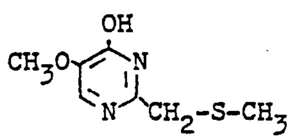

Zu einer Mischung aus 10,4 g (0,1 Mol) Methoxyessigsäuremethylester und 6 g (0,1 Mol) Ameisensäuremethylester gibt man bei 20-25 °C portionsweise 5,4 g (0,1 Mol) festes Natriummethylat und läßt das Gemisch 5 Stunden bei Raumtemperatur nachrühren. Dann gibt man weitere 5,4 g (0,1 Mol) Natriummethylat in 40 ml Methanol gelöst zu, versetzt anschließend das Gemisch mit 14,1 g (0,1 Mol) Methylenmercaptoacetamidin-Hydrochlorid und rührt es 24 Stunden bei Raumtemperatur nach. Jetzt wird das Lösungsmittel im Vakuum abgezogen, der Rückstand in 300 ml Wasser gelöst und die Lösung durch Zugabe von konz. Salzsäure auf pH 6 gebracht. Dann schüttelt man zweimal mit je 150 ml Methylenchlorid aus, trocknet die organische Phase über Natriumsulfat und destilliert das Lösungsmittel im Vakuum ab. Man erhält auf diese Weise 13 g (70 % der Theorie) 2-Methylthiomethyl-6-hydroxy-5-methoxypyrimidin in Form eines hellbraunen Pulvers mit dem Schmelzpunkt 102 °C.

Beispiel b

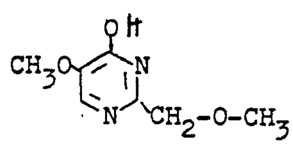

Ein Gemisch aus 17,5 g (0,1 Mol) 2-Chlormethyl-6-hydroxy-5-methoxypyrimidin (Darst. s.u.), 11,7 g (0,22 Mol) Natriummethylat und 150 ml Methanol wird 8 Stunden unter Rückfluß gekocht. Dann bringt

man die gekühlte Mischung durch Zugabe von Salzsäure auf pH 5-6, destilliert das Lösungsmittel im Vakuum ab und verreibt den Rückstand mit 50 ml Äthanol.

Die so erhaltene Suspension wird filtriert, das Filtrat dampft man im Vakuum ein. Zurück bleiben 12, 1 g (71 % der Theorie) 2-Methoxymethyl-6-hydroxy-5-methoxy-pyrimidin mit dem Schmelzpunkt 87 °C.

Das als Ausgangsmaterial zu verwendende 2-Chlormethyl-6-hydroxy-5-methoxypyrimidin kann z. B. wie folgt hergestellt werden :

Zu einer Lösung von 10,8 g (0,2 Mol) Natriummethylat in 500 ml Methanol gibt man bei 0-5 °C 151, 2 g (2 Mol) Chloracetonitril und dann bei 15-20 °C 117,6 g (2,2 Mol) Ammoniumchlorid und rührt das Gemisch 4 Stunden bei Raumtemperatur nach. Danach wird es bei 0-5 °C zu einer Mischung aus 208 g (2 Mol) Methoxyessigsäuremethylester, 120 g (2 Mol) Ameisensäuremethylester und 108 g (2 Mol) festem Natriummethylat, die zuvor 5 Stunden bei Raumtemperatur gerührt wurde, gegeben. Anschließend gibt man bei 0-5 °C eine Lösung von 108 g (2 Mol) Natriummethylat in 400 ml Methanol zu und rührt das Reaktionsgemisch 24 Stunden bei 0-5 °C nach. Unter weiterer Kühlung wird durch Zugabe von konz. Salzsäure der pH-Wert auf 5 eingestellt und dann soviel Wasser zugegeben, daß eine klare Lösung entsteht. Man destilliert das Methanol im Vakuum aus dem Reaktiongemisch, kühlt die zurückbleibende wäßrige Lösung und saugt dann das ausgefallene Produkt ab. Man erhält auf diese Weise 140 g (40 % der Theorie) 2-Chlormethyl-6-hydroxy-5-methoxypyrimidin in Form eines sandfarbenen Pulvers mit dem Schmelzpunkt 188 °C (Z).

Analog einem der Beispiele a oder b können die folgenden Verbindungen der Formel

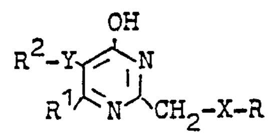

hergestellt werden

| Beispiel | R | $R^1$ | $R^2$ | X | Y | Ausb. (% d.Th.) | Schmelzpunkt (°C) |
|---|---|---|---|---|---|---|---|
| c | $C_2H_5$ | H | $CH_3$ | S | O | 76 | 151 |
| d | $C_3H_7$ | H | $CH_3$ | S | O | 28 | 119 |
| e | $CH_3$ | H | $C_2H_5$ | S | O | 25 | 177(Z)* |
| f | $CH_3$ | H | $CH_3$ | S | S | | |
| g | $CH_3$ | H | i-$C_3H_7$ | S | O | | |
| h | $CH_3$ | H | $C_2H_5$ | S | S | | |
| i | $CH_3$ | $CH_3$ | $CH_3$ | S | S | | |
| j | $CH_3$ | $C_2H_5$ | $CH_3$ | S | S | | |
| k | $CH_3$ | t,-$C_4H_9$ | $CH_3$ | S | S | | |
| l | i-$C_3H_7$ | H | $CH_3$ | S | O | | |
| m | $CH_3$ | H | $C_3H_7$ | S | O | | |
| n | $C_2H_5$ | H | $C_2H_5$ | S | O | | |

*HCl-Salz

**Ansprüche**

1. N,N-Dimethyl-O-pyrimidinyl-carbaminsäureester der Formel I

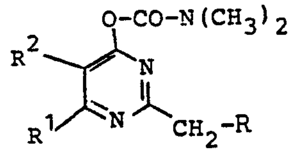

(I)

in welcher

R und $R^2$ gleich oder verschieden sind und einzeln für Alkoxy, Alkylthio, Alkylsulfinyl oder Alkylsulfonyl mit jeweils 1-6 C-Atomen stehen, und

$R^1$ für Wasserstoff oder Alkyl mit 1-6 C-Atomen steht.

2. Verfahren zur Herstellung der N,N-Dimethyl-O-pyrimidinylcarbaminsäureester der Formel I

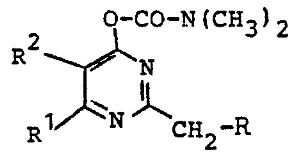

(I)

in welcher

R und $R^2$ gleich oder verschieden sind und einzeln für Alkoxy, Alkylthio, Alkylsulfinyl oder Alkylsulfonyl mit jeweils 1-6 C-Atomen stehen, und

$R^1$ für Wasserstoff oder Alkyl mit 1-6 C-Atomen steht,

dadurch gekennzeichnet, daß man

a) Hydroxy-pyrimidine der Formel II

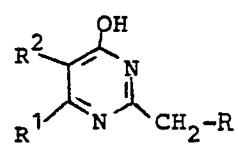

(II)

in welcher R, $R^1$ und $R^2$ die oben angegebene Bedeutung haben,

mit N,N-Dimethyl-carbaminsäurehalogeniden der Formel III

$$Hal—CO—N(CH_3)_2 \qquad (III)$$

in welcher Hal für Chlor oder Brom steht,

gegebenenfalls in Gegenwart eines Säureakzeptors und gegebenenfalls unter Verwendung eines Verdünnungsmittels umsetzt, oder

b) Hydroxy-pyrimidine der Formel II, worin R, $R^2$ und $R^1$ die oben angegebene Bedeutungen haben, mit Phosgen und anschließend mit Dimethylamin, gegebenenfalls in Gegenwart eines Säureakzeptors und gegebenenfalls unter Verwendung eines Verdünnungsmittels umsetzt oder

für den Fall, daß in Verbindungen der Formel I R und/oder $R^2$ für Alkylsulfinyl steht,

c) N,N-Dimethyl-O-pyrimidinyl-carbaminsäureester der Formel I, in welcher R und/oder $R^2$ für Alkylthio steht, mit der äquimolaren Menge Wasserstoffperoxid, gegebenenfalls unter Verwendung eines Verdünnungsmittels umsetzt, oder

für den Fall, daß in Verbindungen der Formel I R und/oder $R^2$ für Alkylsulfonyl steht,

d) N,N-Dimethyl-O-pyrimidinyl-carbaminsäureester der Formel I, in welcher R und/oder $R^2$ für Alkylthio steht, mit wenigstens zwei Moläquivalenten m-Chlorperbenzoesäure, gegebenenfalls in Gegenwart eines Verdünnungsmittels umsetzt.

3. Schädlingsbekämpfungsmittel, gekennzeichnet durch einen Gehalt an mindestens einem N,N-Dimethyl-O-pyrimidinyl-carbaminsäureester der Formel I.

4. Verwendung von N,N-Dimethyl-O-pyrimidinyl-carbaminsäureestern der Formel I zur Bekämpfung von Schädlingen.

5. Verfahren zur Herstellung von Schädlingsbekämpfungsmitteln, dadurch gekennzeichnet, daß man N,N-Dimethyl-O-pyrimidinyl-carbaminsäureester der Formel I mit Streckmitteln und/oder oberflächenaktiven Mitteln vermischt.

**Claims**

1. N,N-Dimethyl-carbamic acid O-pyrimidinyl esters of the formula I

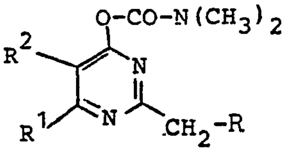

(I)

in which

R and $R^2$ are identical or different and individually represent alkoxy, alkylthio, alkylsulphinyl or alkylsulphonyl with in each case 1-6 C atoms, and

$R^1$ represents hydrogen or alkyl with 1-6 C atoms.

2. Process for the preparation of the N,N-dimethylcarbamic acid O-pyrimidinyl esters of the formula I,

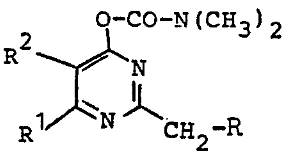

(I)

in which

R and $R^2$ are identical or different and individually represent alkoxy, alkylthio, alkylsulphinyl or alkylsulphonyl with in each case 1-6 C atoms, and

$R^1$ represents hydrogen or alkyl with 1-6 C atoms,

characterised in that

a) hydroxy-pyrimidines of the formula II

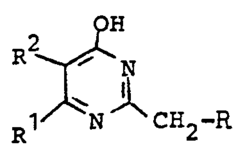

(II)

in which R, $R^1$ and $R^2$ have the meaning indicated above, are reacted with N,N-dimethyl-carbamic acid halides of the formula III

$$Hal—CO—N(CH_3)_2 \qquad (III)$$

in which Hal represents chlorine or bromine,

if appropriate in the presence of an acid acceptor and if appropriate using a diluent, or

b) hydroxy-pyrimidines of the formula II, wherein R, $R^2$ and $R^1$ have the meaning indicated above, are reacted with phosgene and then with dimethylamine, if appropriate in the presence of an acid acceptor and

if appropriate using a diluent, or in the case where, in compounds of the formula I, R and/or $R^2$ represents alkylsulphinyl

c) N,N-dimethyl-carbamic acid O-pyrimidinyl esters of the formula I in which R and/or $R^2$ represents alkylthio are reacted with an equimolar amount of hydrogen peroxide,

if appropriate using a diluent, or in the case where, in compounds of the formula I, R and/or $R^2$ represents alkylsulphonyl,

d) N,N-dimethyl-carbamic acid O-pyrimidinyl esters of the formula I in which R and/or $R^2$ represents alkylthio are reacted with at least two molar equivalents of m-chloroperbenzoic acid, if appropriate in the presence of a diluent.

3. Agents for combating pests, characterised in that they contain at least one N,N-dimethyl-carbamic acid O-pyrimidinyl ester of the formula I.

4. Use of N,N-dimethyl-carbamic acid O-pyrimidinyl esters of the formula I for combating pests.

5. Process for the preparation of agents for combating pests, characterised in that N,N-dimethyl-carbamic acid O-pyrimidinyl esters of the formula I are mixed with extenders and/or surface-active agents.

**Revendications**

1. Esters d'acides N,N-diméthyl-O-pyrimidinyl-carbamiques de formule I

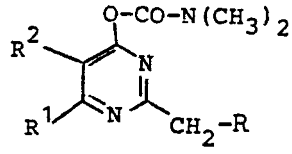

(I)

dans laquelle

R et $R^2$ sont identiques ou différents et représentent chacun un groupe alcoxy, un groupe alkylthio, un groupe alkylsulfinyle ou un groupe alkylsulfonyle contenant chacun 1 à 6 atomes de carbone, et

$R^1$ représente un atome d'hydrogène ou un groupe alkyle contenant 1 à 6 atomes de carbone.

2. Procédé de préparation des esters d'acides N,N-diméthyl-O-pyrimidinyl-carbamiques de formule I

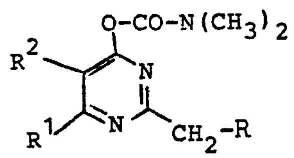

(I)

dans laquelle

R et $R^2$ sont identiques ou différents et représentent chacun un groupe alcoxy, un groupe alkylthio, un groupe alkylsulfinyle ou un groupe alkylsulfonyle contenant chacun 1 à 6 atomes de carbone, et

$R^1$ représente un atome d'hydrogène ou un groupe alkyle contenant 1 à 6 atomes de carbone, caractérisé en ce que :

a) on fait réagir des hydroxy-pyrimidines de formule II

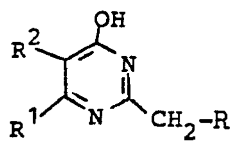

(II)

dans laquelle R, $R^1$ et $R^2$ ont les significations indiquées ci-dessus, avec des halogénures d'acides N,N-diméthyl-carbamiques de formule III

$$Hal—CO—N(CH_3)_2 \quad (III)$$

dans laquelle Hal représente un atome de chlore ou un atome de brome,
éventuellement en présence d'un accepteur d'acide et en utilisant éventuellement un diluant, ou

b) on fait réagir des hydroxy-pyrimidines de formule II dans laquelle R, $R^2$ et $R^1$ ont les significations indiquées ci-dessus, avec du phosgène, puis avec la diméthylamine, éventuellement en présence d'un accepteur d'acide et en utilisant éventuellement un diluant, ou
si, dans les composés de formule I, R et/ou $R^2$ représente(nt) chacun un groupe alkyl-sulfinyle,

c) on fait réagir des esters d'acides N,N-diméthyl-O-pyrimidinyl-carbamiques de formule I dans laquelle R et/ou $R^2$ représente(nt) chacun un groupe alkylthio,
avec une quantité équimolaire de peroxyde d'hydrogène en utilisant éventuellement un diluant, ou si,, dans les composés de formule I, R et/ou $R^2$ représente(nt) chacun un groupe alkyl-sulfonyle,

d) on fait réagir des esters d'acides N,N-diméthyl-O-pyrimidinyl-carbamiques de formule I dans laquelle R et/ou $R^2$ représente(nt) chacun un groupe alkylthio, avec au moins deux équivalents molaires d'acide m-chloroperbenzoïque, éventuellement en présence d'un diluant.

3. Agent parasiticide, caractérisé en ce qu'il contient au moins un ester d'acide N,N-diméthyl-O-pyrimidinyl-carbamique de formule I.

4. Utilisation d'esters d'acides N,N-diméthyl-O-pyrimidinyl-carbamiques de formule I pour combattre les parasites.

5. Procédé de préparation d'agents parasiticides, caractérisé en ce qu'on mélange des esters d'acides N,N-diméthyl-O-pyrimidinyl-carbamiques de formule I avec des diluants et/ou des agents tensio-actifs.